# EUROPEAN PATENT APPLICATION

(11) **EP 4 455 162 A1**
(43) Date of publication of application: **30.10.2024**
(21) Application number: 22850571.5
(22) Date of filing: 09.12.2022
(51) Int. Cl.: C07K 16/28, C07K 16/30

(54) **FUSION PROTEINS COMPOSED OF AN ANTIBODY AND A MUTEIN**

(30) Priority: 21.12.2021 CU 20210104
(71) Applicant: Centro de Inmunologia Molecular, Playa, La Habana 11300 (CU)
(72) Inventor: CASADESÚS PAZOS, Ana Victoria, La Habana 10400 (CU); HERNÁNDEZ GARCÍA, Tays, La Habana 11300 (CU); LEÓN MONZÓN, Kalet, La Habana 17100 (CU)
(74) Representative: V.O.
(86) International application number: PCT/CU2022/050012
(87) International publication number: WO 2023/116951

(57) **Abstract**

The present invention is in the field of Biotechnology and Immuno-oncology. Fusion proteins are described that comprise an interleukin 2 agonist mutein linked to an immunoglobulin via a linker. These fusion proteins are useful in the treatment of cancer given their superior properties to other similars based on non-alpha IL-2 mutein, by simultaneously preserving the ability of the antibodies to effect ADCC and CDC and also activate NK and TCD8 + cells, without expanding regulatory T cells. The convergence of these properties results in fusion proteins with antitumor properties superior to the parental antibodies, and even to the combination of these with non-alpha mutein.

## Description

### FIELD OF THE INVENTION

The present invention relates to the field of Biotechnology and Immuno-oncology. Particularly, it describes fusion proteins composed of no alpha muteins agonists of interleukin 2 (IL-2) fused to antibodies specific for antigens expressed on the tumor, and capable to recruit effector functions.

### BACKGROUND

Immunocytokines (ICs), biopharmaceutical products composed of a cytokine fused to an antibody or an antibody fragment, are attractive immunotherapeutic tools for the treatment of cancer, although their use has been extended to other diseases.

IL-2 is a potent activator of T lymphocytes and stimulator of NK cells and their ADCC (Antibody Dependent Cellular Cytotoxicity) (Hank et al. (1990) J Biol Response Mod. 9:5-14). For this reason, it has become one of the most used cytokines in the generation of this kind of fusion proteins. Indeed, most of clinical progress have been made with those ones that fusion IL-2 to antibodies specific for tumor antigens (Sondel, P. M. and S. D. Gillies. (2012) Antibodies. 1(2): 149-171). Despite the promising results in Phase II clinical trials (Albertini, M. R. et al. (2012) Cancer Immunol Immunother. 61(12): 2261-2271; Danielli, R., et al. (2015) Cancer ImmunolImmunother. 64(8): 999-1009; Kaufman, L. (2014) Journal of ClinicalOncology. 32(15); Lansigan, F. et al. (2016) Blood. 128 (22): 620), these ICs keep adverse effects similar to that of recombinant IL-2, like the high toxicity profile (Panelli, M. C. et al. (2004) J Transl Med. 2(1): 17; Skrombolas, D. and J. G. Frelinger. (2014) Expert Rev Clin Immunol. 10(2): 207-217; Klein, C., et al. (2017) Oncoimmunology. 6(3): e1277306) and the expansion of regulatory T cells, potentially able to inhibit the antitumor activity (Ahmadzadeh, M. and S. A. Rosenberg. (2006) Blood. 107(6): 2409-2414; Jensen, H. K., et al. (2009) Clin Cancer Res. 15(3): 1052-1058; Fournier, P., et al. (2011) Int J Oncol. 38(6): 1719-1729; Gubbels, J. A., et al. (2011) Cancer Immunol Immunother. 60(12): 1789-1800; Pretto, F., et al. (2014) Cancer Immunol Immunother. 63(9): 901-910; Sim, G. C., et al. (2014) J Clin Invest. 124(1): 99-110; Lansigan, F. et al. (2016) Blood. 128 (22):620). An additional limitation is associated to a suboptimal biodistribution and targeting to the tumor because of the interaction with the high affinity IL-2 receptors (Klein, C., et al. (2017) Oncoimmunology. 6(3): e1277306; Waldhauer, I., et al. (2021) Mabs. 13(1): 1913791). This scenario has stimulated the development of ICs based on mutated variants of IL-2 (no alpha muteins), rationally designed to have a reduced or null capability to bind the alpha chain of IL-2 receptor, and in turn, to stimulate specific subsets of immune cells, as well as diminishing toxicity (Runbeck, E., et al. (2021) Antibodies. (Basel) 10(1)).

Among these ICs there is a group that includes those ones based on IL-2v, characterized by the presence of mutations F42A, Y45A, L72G, which abrogates the binding to alpha chain of IL-2 receptor and favors the expansion of effector CD8+T and NK cells, and no preferential proliferation of regulatory T cells. These fusion proteins have a full-length IgG (IgG1) format, do not possess classic antibody effector functions such as CDC (Complement Dependent Cytotoxicity) and ADCC, while being monovalent or monofunctional regarding the cytokine. Within this group is the IC cergutuzumab amunaleukin (CEA-IL2v), which consists of a specific antibody for the carcinoembryogenic antigen (CEA) fused through the C-terminus of one of its heavy chains to one molecule of the IL-2v mutant (Klein, C., y cols. (2017) Oncoimmunology. 6(3): e1277306).

In this set are also included the ICs Simlukafusp alfa (FAP-IL2v), specific for the fibroblast activation protein α (Waldhauer, I., et al. (2021) Mabs. 13(1): 1913791) and the IC anti -PD1-IL2v (Klein, C., et al. (2019) Cancer Res. 79(13 Suppl): Abstractnr 1552), which recognizes the programmed death 1 (PD-1) molecule (Klein, C., et al. (2019) Cancer Res. 79(13 Suppl): Abstractnr 1552). On the other hand, it has been reported the IC Erb-Sum-IL2, specific for EGFR (epidermal growth factor receptor), which contains a variant of IL-2 (sum-IL-2) characterized by mutations L80F, R81D, L85V, I86Y, I92F. This set of changes with respect to the wild type cytokine determines a reduced binding and greater interaction of the mutein with the alpha and beta chains of the receptor, respectively. This conditions favorable binding to activated CD8+ T cells and not to regulatory T cells. This IC is presented as a heterodimeric Fc fusion protein containing a sum-IL2 monomer on one arm and an anti-EGFR Fab on the other one. For this monovalent IC with respect to the antigen-binding site and with respect to the cytokine, a preferential expansion of CD8+ T cells as compared to regulatory T cells in the tumor microenvironment has been described, while the ability to recruit antibody classic effector functions such as ADCC and CDC has not been reported (Sun, Z., et al. (2019) Nat Commun. 10(1): 3874).

Considering the previously described background information, the inventors of the present application generated a type of IC for cancer therapy, whose design is based on the fusion of an IL-2 agonist mutein (IL2non-alpha) to the carboxyl terminus of the heavy chains of an IgG antibody with the capacity to recruit antibody effector functions, to render a bifunctional molecule with respect to the antigen binding site and the cytokine. Surprisingly, this type of molecule has superior properties to other similar ones based on non-alpha muteins, by conserving or simultaneously improving the ability of antibodies to carry out direct lysis, ADCC and/or CDC and, in addition, activating NK and CD8+ T cells, without expanding regulatory T cells. The convergence of these properties results in fusion proteins with antitumor properties superior to the parental antibodies, and even to their combination with IL2non-alpha. Finally, the format and composition of the fusion proteins listed in the present invention do not interfere with the biological activities associated with the variable region of the antibody portion. Such is the case of the capacity to induce apoptosis or to induce cytotoxicity independent of effector functions.

### BRIEF DESCRIPTION OF THE INVENTION

In one embodiment, the subject of the present invention is a fusion protein characterized by comprising an IL-2 agonist mutein (non-alpha mutein) bound to an immunoglobulin of the IgG1 or IgG3 isotype, and said immunoglobulin is recognized by Fc gamma receptors and complement cascade molecules. This immunocytokine is characterized by being a bivalent molecule with respect to the antigen binding site and the cytokine. Such a mutein and said immunoglobulin are connected through a linker. Particularly, the mutein's ability to bind to the alpha chain of the IL-2 receptor is affected by at least two orders of magnitude, and the mutein binds to immunoglobulin through the carboxy-terminus of each heavy chain of said immunoglobulin. In particular, the non-alpha mutein has a sequence that is selected from the group comprising SEQ ID NO. 16-43.

In a particular embodiment, the linker is composed of the amino acid sequence that is selected from the group comprising: (Gly₄Ser)ₙThrGly (SEQ ID NO. 44) and (Gly₄Ser)ₙ (SEQ ID NO. 45), where n is the number of repeats of the Gly₄Ser fragment and has a value from 1 to 5.

In a particular embodiment, the immunoglobulin heavy chain belongs to the human IgG1 or IgG3 subclasses and comprises the sequences SEQ ID NO. 11 or 12 or has higher than 97% identity with sequences 11 or 12.

In a particular embodiment, the light chain of the immunoglobulin is characterized by belonging to the human kappa isotype and has a sequence that corresponds to SEQ ID NO. 9.

In a particular embodiment, the light chain of the immunoglobulin is characterized by belonging to the human lambda isotype and has a sequence that corresponds to SEQ ID NO. 10.

The immunocytokine of the present invention is further characterized by recognizing tumor antigens among which are included: CD20, CD19, NGcGM3, PDL1, Her1, Her2 and Epcam.

In an additional embodiment, the present invention relates to pharmaceutical compositions that comprise the fusion proteins described herein in a concentration range of 0.5 mg/ml to 20 mg/ml and a pharmaceutically acceptable vehicle.

Additionally, the present invention relates to the use of the fusion proteins disclosed in the present invention in the treatment of cancer. In particular, with the use of the nucleic acid molecules, encoding the fusion proteins of the present invention, for subcutaneous, intramuscular or intratumoral injection. In an additional embodiment, the present invention relates to a method of treatment for a subject in need, that comprises the subcutaneous, intravenous, intradermal, intramuscular or intraperitoneal administration of the pharmaceutical compositions of the present invention in a dose range between 0.01 mg/Kg - 0.6 mg/Kg of weight. Particularly, the administration of said pharmaceutical compositions is carried out from one to 30 cycles between one and three times a week and the time between the cycles is between seven days and eight weeks.

### DETAILED DESCRIPTION OF THE INVENTION

### Description of the ICs

In one embodiment, the object of the present invention is fusion proteins or ICs based on an IL-2 agonist non-alpha mutein (non-alpha mutein), which have at least two orders of reduced binding affinity to the alpha chain of the receptor. (Leon et al. (2018) Semin Oncol. 45(1-2): 95-104), bound to the heavy chains of an immunoglobulin with the capacity to recruit classic effector functions of antibodies, to render a bifunctional molecule with respect to the antigen binding site and the cytokine.

The term "fusion protein" as used in the present invention refers to a polypeptide that comprises an amino acid sequence of an antibody and an amino acid sequence of a heterologous polypeptide or protein, that is, a polypeptide or protein that is not normally part of the antibody, connected through a peptide linker.

The term immunocytokine (IC) as used herein refers to an antibody format which is bound to a cytokine. Such a cytokine is fused to the antibody by means of a linker at the carboxy terminus of the heavy chain.

The terms "fusion protein" and "IC" are used interchangeably in the present invention.

The muteins forming part of the ICs described in the present invention are affected by at least two orders of magnitude, their binding capacity to the high-affinity receptor constitutively expressed in regulatory T cells, specifically, to the alpha chain. The fusion proteins of the present invention exhibit a property that other non-alpha mutein-based ICs do not have: the ability to activate NK and CD8+ T without expanding regulatory T, while recruiting effector functions such as direct lysis, ADCC and/or CDC. These muteins will be referred to herein as non-alpha mutein.

Particularly, the IC antibody portion is a human IgG1 or IgG3 subclass immunoglobulin whose amino acid sequence corresponds to natural or modified allelic variants of IgG1 or IgG3 having more than 97% identity with those identified as SEQ ID NO. 11 and SEQ ID NO. 13, respectively, and that bind to Fcy receptors and complement molecules. Therefore, they are compatible with the activities of ADCC and CDC. The constant region of the light chain of the antibodies forming part of the fusion proteins of the present invention is of the human kappa or lambda isotype and their sequences are described in SEQ ID NO. 9 and SEQ ID NO. 10. On the other hand, the variable regions of the antibody portion can be murine, humanized or completely human and come from immunoglobulins with the capacity to make ADCC and CDC. The variable regions of the antibody portion of the ICs of the present invention recognize antigens expressed in human tumors, such as: CD20, CD19, ganglioside NGcGM3, PDL1, Her1, Her2, and Epcam.

The non-alpha muteins described in the present invention are attached to the carboxyl terminus of each antibody heavy chain through a flexible linker peptide (Gly₄Ser)ₙThrGly (SEQ ID NO. 44) or (Gly4Ser)n (SEQ ID NO 45), where n is the number of repeats of the Gly4Ser fragment, and have the ability to preferentially expand CD8+ and NK effector T cells, in detriment of the expansion of regulatory T cells.

Non-alpha muteins can be any of those described in SEQ ID NO. 16-43, previously disclosed in US 9,206,243 B2 and US patent 2019/0315826 A1, or IL-2v, previously disclosed in patent WO2012107417 (SEQ ID NO. 40) and its variants described in SEQ ID NO 41-43. The muteins described in SEQ ID NO. 16-21, generated at the Center for Molecular Immunology, are IL-2 agonists and their binding capacity to the high-affinity receptor constitutively expressed in regulatory T cells is affected in at least two orders. As a result of their modifications with respect to wild type IL-2, they are able to preferentially expand effector populations of NK and memory CD8+ T cells, have an agonist action of native IL-2, and have a greater antitumor effect than native IL-2 in animal models (US 9,206,24309). The IL2non-alpha K35E, IL2non-alpha K35Q and IL2non-alpha K35D muteins (SEQ ID NO. 22-39) are variants of the IL2non-alpha obtained with the method described in US patent 9,206,243, which contain a point mutation that favors expression in different host systems and is compatible with the interaction profile of the non-alpha mutein with the different chains of the IL-2 receptor (Rojas, G., et al. (2015) J Mol Recognit. 28(4): 261-268). The IL-2v mutant and its variants (SEQ ID NO. 40-43) contain mutations with respect to wild type IL-2 that prevent binding to the alpha chain of the receptor, which prevents the preferential expansion of regulatory T cells and favors the expansion of effector TCD8+ and NK cells (Klein, C., et al. (2017) Oncoimmunology. 6(3): e1277306).

In summary, the format of the fusion proteins of the present invention guarantees the maintenance or improvement of the classic effector functions of antibodies (direct lysis, ADCC and CDC), which coincide with the particular immunomodulatory properties of non-alpha muteins, consisting of induction of preferential proliferation of effector cells with respect to regulatory T cells.

### Pharmaceutical compositions

The ICs object of the present invention can be found as an active ingredient, forming part of different appropriate pharmaceutical compositions for the same, and a pharmaceutically acceptable vehicle. Concentrations of the active ingredient in said pharmaceutical compositions are in the range of 0.5 mg/ml to 20 mg/ml, preferably 1 mg/ml to 10 mg/ml, or lyophilized. Pharmaceutically acceptable vehicles include, but are not limited to: saline, pH neutral phosphate buffered saline, and others alike. Other buffering agents, dispersing agents, and non-toxic inert substances suitable for delivery to a patient may be included in the compositions of the present invention. The compositions may be solutions suitable for administration, and are normally sterile and free of undesirable particles.

### Therapeutic Use and Treatment

The novelty of the ICs described in the present invention consists of the convergence in a bifunctional fusion protein based on an IgG1 or IgG3 immunoglobulin and a non-alpha mutein, of the ability to preferentially expand and activate effector cells such as memory TCD8+ and NK over regulatory T cells, and to maintain or enhance effector functions typical of antibodies, such as direct lysis, ADCC, and CDC. This concurrency has not been described for any of the ICs based on non-alpha muteins described to date. In addition, the presence of two non-alpha IL-2 mutein molecules per IC molecule makes it possible to increase the intrinsic immunomodulatory activity of the fusion protein. This further contributes to a potential decrease in toxicity due to the non-interaction with high affinity receptors on endothelial cells. The Fc region offers the possibility of obtaining them highly purified by affinity chromatography with protein A, among others, which allows them to be administered as a soluble protein by different routes (subcutaneous, intravenous, intradermal, intramuscular, intraperitoneal), while increasing the half-life time of these agents in circulation and, in turn, the therapeutic effectiveness.

The ICs of the present invention will be applied to subjects (these subjects are vertebrates, such as humans), preferably subcutaneously, or intravenously, in a dose range between 0.01 mg/Kg of weight and 0.6 mg/Kg of weight, which correspond to 0.7 and 42 mg total, respectively. The IC will be administered to the patient for one to 30 cycles with a frequency of one to three times per week. The time between administration cycles will be from seven days to eight weeks. Additionally, the delivery strategies employed in the present invention for these ICs may also include injection of the product, through gene therapy approaches such as injection of mRNA and transducing particles encoding them.

Given the immunomodulatory properties of these molecules, in addition to the potentiation of the antigen-specific antitumor response, the systemic immunostimulation capacity supports the possible combination with therapies directed to other targets in patients with various types of cancer. In the same way, its combination with other immunomodulators and with classic oncotherapies such as chemotherapy and radiotherapy is also possible. In particular, this type of biopharmaceutical could be used in the treatment of B-cell lymphoproliferative disorders, such as non-Hodgkin lymphoma (NHL), chronic lymphocytic leukemia (CLL), diffuse large B-cell lymphoma (DLBCL), Burkitt lymphoma. (BL), Mantle Cell Lymphoma (MCL), Follicular Lymphoma, Indolent Lymphomas, Marginal Zone Lymphoma (MZL), Critical Lymphoplasmacytic Lymphoma (PL), B-Cell Lymphoproliferative Syndrome, Advanced Stage Solid Tumors, Head and Neck Tumors neck, brain tumors, adult and pediatric gliomas, pancreatic cancer, esophagus, non-small cell lung cancer, and nasopharyngeal tumors.

In this way, the aforementioned ICs are intended to constitute a therapeutic front that enhances the protective action of antibodies and IL-2, currently used in the treatment of cancer. This effect would be associated with the generation of fusion proteins that can simultaneously have ADCC and CDC activity, important antitumor mechanisms, and preferentially expand cytotoxic T and NK cells, and not regulatory T cells, leading to a more efficient antitumor immune response, and therefore, to a retardation in tumor growth and a greater survival of the treated individuals. Additionally, the lower levels of toxicity guaranteed by the IL-2 muteins used, increases the probability of success compared to therapies with wild type cytokines. All of this translates into greater life expectancy and quality of life in treated patients.

### BRIEF DESCRIPTION OF THE FIGURES

**Figure 1****.** Representation of ICs IgG-IL2non-alpha. (A) Scheme (B) Format.
**Figure 2****.** Evaluation of the expression of the ICs in the supernatant of transduced CHO cells (A, E, F) and in HEK293T cells in suspension transiently transfected (B, C, D).
**Figure 3****.** Sodium dodecyl sulfate polyacrylamide gel electrophoresis (SDS-PAGE) of anti-CD20-IgGlh-IL2non-alpha (IC1) under non-reducing (A) and reducing (B) conditions.
**Figure 4****.** Immunoidentification of IC1 and simple controls by Western blot, with a specific antibody for human IgG, under non-reducing (A) and reducing (B) conditions and with a specific antibody for IL-2 under non-reducing conditions (C).
**Figure 5****.** Size exclusion-high-performance liquid chromatography (SEC-HPLC) analysis of IC1(A) and rituximab (B) on a TSK gel 3000 column.
**Figure 6****.** SDS-PAGE of IC anti-CD20-IgG2a-IL2non-alpha (IC7) under non-reducing (A) and reducing (B) conditions.
**Figure 7****.** Immunoidentification of IC7 and simple controls by Western blot, with a specific antibody for IL-2, under non-reducing conditions.
**Figure 8****.** SEC-HPLC analysis of IC7 on a TSKgel 3000 column.
**Figure 9****.** SDS-PAGE of IC anti-NGcGM3-IL2non-alpha (IC8) under non-reducing (A) and reducing (B) conditions.
**Figure 10****.** Immunoidentification of IC8 and the simple controls by Western blot, with a specific antibody for IL-2, under non-reducing conditions.
**Figure 11****.** SEC-HPLC analysis of IC8 (1) and 14F7m (2) on a TSKgel 3000 column.
**Figure 12****.** Immunoidentification of IC4 and simple controls by Western blot, with a specific antibody for human IgG, under non-reducing conditions.
**Figure 13****.** Recognition of the EL4-huCD20+ cell line by the RTX antibody and IC1 (A) and by the mouse RTX-IgG2a antibody and IC7(B).
**Figure 14****.** Recognition of the CD20 molecule on tumor cells by IC1 (A). Ratio of mean fluorescence intensity (MFI) of RTX and IC1 over the MFI of the conjugate (B).
**Figure 15****.** CDC induction by IC1.
**Figure 16****.** Induction of apoptosis by IC1. (A) Early apoptosis, (B) Apoptosis (sum of all Annexin V+ cells) and (C) caspase 3 activation, in IC1-treated Ramos cells.
**Figure 17****.** Induction of ADCC by IC1 (A). In vitro depletion of B cells (CD19+) from a healthy donor (B) and a patient with diffuse large B-cell lymphoma (C) by IC1.
**Figure 18****.** Recognition of the NGcGM3 ganglioside by IC8 as measured by (A) ELISA and (B) flow cytometry.
**Figure 19****.** Induction of complement-independent cytotoxicity by IC8.
**Figure 20****.** IL-2non-alpha type activity of (A) IC1 and (B) IC7 and IC8, as measured by proliferation assay of the CTLL-2 cell line. The concentration values are referred to the non-alpha IL-2 portion contained in each sample.
**Figure 21****.** Measurement of surface markers of NK cell activation induced by IC1, (A) CD16, (B) CD69 and (C) CD107a.
**Figure 22****.** Antitumor effect of IC7 in the EL4-huCD20 model. (A) Comparison with the parental antibody at high doses. (B) Comparison with the combination of the parental antibody and the IL2non-alpha mutein (in equimolar amounts).
**Figure 23****.** Antitumor effect of IC1 in the EL4-huCD20 model: (A) Comparison with high-dose rituximab, (B) Comparison with equimolar amounts of rituximab and (C) Long-lasting protective effect of IC1 in surviving animals challenged again with tumor cells. Antitumor effect of IC8 with respect to equimolar amounts of the antibody and the IL2non-alpha mutein in the 3LL-D122 model (D).
**Figure 24****.** In vivo immunomodulation by IC1 in C57BL/6 mice bearing EL4-hCD20 tumor. (A-B) Absolute numbers of (A) CD8+ T cells, (B) NK1.1+CD3-cells, (C-D) Proportion of cells (C) FOXP3+CD4+/CD8+, (D) FOXP3+CD4+/NK1.1+ CD3+ and (E) absolute numbers of FOXP3+CD4+ T cells.

The present invention is further elaborated by the following examples and drawings. However, these examples should not be construed as limiting the scope of the invention.

### EXAMPLES

### Example 1. Design and obtaining of the ICs.

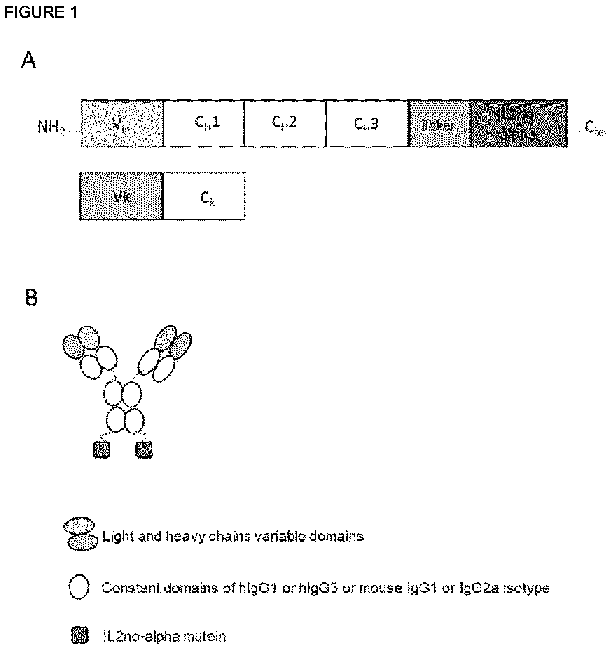

Human ICs consisting of bivalent molecules with respect to the antigen binding site, bifunctional with respect to the mutated IL-2 portion and based on a full-length IgG format were designed. Specifically, IC1, IC2, IC3, IC4, IC5 and IC6 correspond to a format like that represented in Figure 1 (A-B).

IC1 and IC2 are specific for the human CD20 molecule and the sequences of their variable regions are those of rituximab, a leading monoclonal antibody in passive therapy against B-cell lymphoproliferative disorders (Pierpont, T. M., et al. (2018) Front Oncol 8: 163; Sehn, L. H. & G. Salles. (2021) N Engl J Med. 384(9): 842-858). The light chain of each of these ICs is formed by a VL variable region (SEQ ID NO. 1) and a human C kappa constant region (SEQ ID NO. 9). The heavy chain is constituted by the VH variable region of sequence SEQ NO. 2, followed by a human IgG1 constant region (SEQ ID NO. 11), which has a linker peptide (Gly₄Ser)3ThrGly (SEQ ID NO. 44) fused to the carboxy terminus, and then an IL2non-alpha molecule (SEQ ID No. 21 for IC1, and SEQ ID NO. 37 for IC2). ICs of this specificity containing mutated variants of IL-2 have not been described to date.

IC3 and IC4 are specific for the NGcGM3 ganglioside, which is absent or detected only in small amounts in normal tissues (Varki, A. (2009) Glycoconj J 26(3): 231-245). The sequences of their variable regions are those of the 14F7hT antibody, currently in a clinical trial for the treatment of advanced-stage solid tumors (EC-IICRDEC168.). The light chain of both ICs is formed by a VL variable region (SEQ ID NO. 3) and a human C kappa constant region (SEQ ID NO. 9). The heavy chain consists of the VH variable region of sequence SEQ ID NO. 4, followed by a human IgG1 constant region (SEQ ID NO. 11) that has fused to the carboxyl terminus a linker peptide (Gly4Ser)3ThrGly (SEQ ID NO. 44) followed by an IL2non-alpha molecule (SEQ ID NO. 21 for IC3, and SEQ ID NO. 37 for IC4). To date, no IC based on this antibody has been obtained, nor any other specific for the NGcGM3 ganglioside.

IC5 and IC6 recognize the EGFR receptor and the sequences of their variable regions correspond to those of the antibody nimotuzumab (US 5,891,996 B2), widely used in the therapy of tumors of epithelial origin (Crombet T, et al. (2002) Int J Cancer 101(6):567-75 Ramos-Suzarte M et al (2012) Cancer Biol Ther 13(8):600-5 Suarez Martinez G and BencomoYanes A. (2014) Biotecnol APL [Internet] 31(2):159-167, Crombet T. (2014) Handbook of therapeutic antibodies. 1679-94.). The light chain is composed of a VL variable region (SEQ ID NO. 5) and a human C kappa constant region (SEQ ID NO. 9). The heavy chain is composed of the VH variable region of sequence SEQ ID NO. 6, followed by a human IgG1 constant region (SEQ ID NO. 11) that has fused to the carboxy- terminus a linker peptide (Gly₄Ser)₃ThrGly (SEQ ID NO. 44) followed by an IL2non-alpha molecule (SEQ ID NO. 21, for IC5, SEQ ID NO. 37 for IC6).

Two other ICs bivalent with respect to the antigen-binding site, and bifunctional with respect to the mutated IL-2 portion, were designed in parallel, based on a complete mouse IgG format (Figure 1A-B).

IC7 is specific for the human CD20 molecule and the sequences of its variable regions are those of rituximab. The light chain is composed of a VL variable region (SEQ ID NO. 1) and a C kappa mouse constant region (SEQ ID NO. 13). The heavy chain is made up of the VH variable region of sequence SEQ ID NO. 2, followed by a constant region of mouse IgG2a (SEQ ID NO. 15) that has a linker peptide (Gly4Ser)3ThrGly (SEQ ID NO. 44) fused to the carboxyl terminus followed by the IL2non-alpha molecule (SEQ ID No. 21).

IC8 is specific for the NGcGM3 ganglioside. The sequences of its variable regions are those of the 14F7 antibody (US 6,429,295 B1), murine ancestor of 14F7hT. The light chain is formed by a VL variable region (SEQ ID NO. 7) and a C kappa mouse constant region (SEQ ID NO. 13). The heavy chain is composed of the VH variable region of sequence SEQ ID NO. 8., followed by a constant region of mouse IgG1 (SEQ ID NO. 14) that has a linker peptide (Gly₄Ser)₃ThrGly (SEQ ID NO. 44) fused to the carboxy terminus and then, an IL2non-alpha molecule (SEQ ID NO.21).

To obtain human ICs, the genes encoding the variable region of the light chain of the rituximab, nimotuzumab or 14F7hT antibodies were cloned into the vector pFUSE (Ckh) containing the human C kappa constant region gene, thus assembling the genes encoding the respective light chains.

Subsequently, the gene segment encoding the IL2non-alpha mutein (SEQ ID NO. 21 or SEQ ID NO. 37) fused through its N-terminus to the linker peptide (Gly₄Ser)₃ThrGly (SEQ ID NO. 44) was amplified by using Polymerase Chain Reaction (PCR), and next cloned EcoRV/XbaI into the vector pFUSE (IgG1h) previously modified for the insertion of an EcoRV site. In the resulting genetic constructs, the corresponding VH gene, previously amplified by PCR, were cloned EcoRI/NheI.

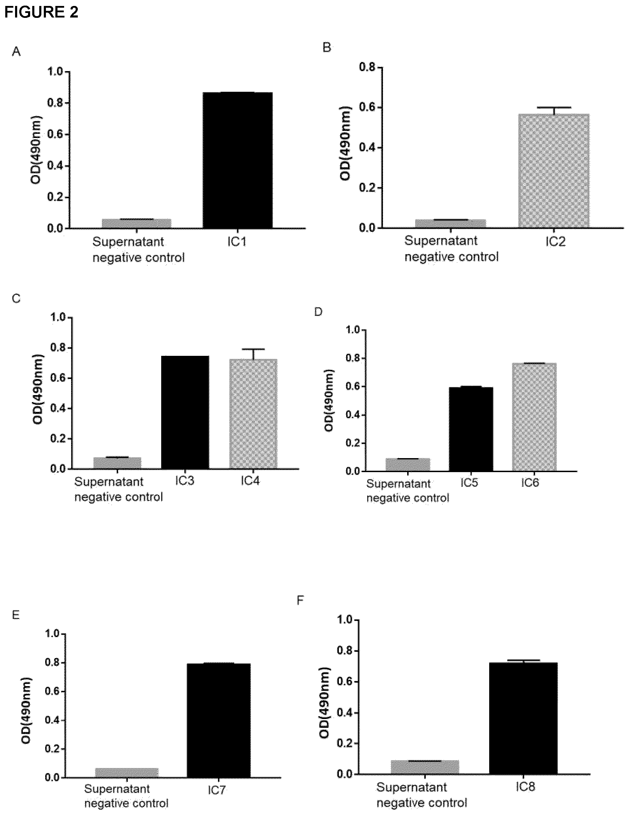

The IC1 heavy and light chain genes (anti-CD20) were cloned independently in the pLV-CMV-IRES-Neo vector, to generate the respective transfer vectors with which we proceeded to obtain the lentiviral transducing particles. CHO-K1 cells were transduced with the previously obtained lentiviral particles for the generation of recombinant stable clones. Using a specific ELISA for the human Fc region, the IC1 fusion protein (anti-CD20) was detected in the supernatant of transduced CHOK1 cells (Figure 2A).

IC2 (anti-CD20) was obtained by transient expression from the plasmid DNA corresponding to the genetic constructs encoding the light and heavy chains in the pFUSE vectors (Figure 2B).

IC3 and IC4 (anti-NGcGM3) were obtained by transient expression using plasmid DNA corresponding to the genetic constructs encoding the heavy and light chains in the pFUSE vectors. HEK293T cells were transiently transfected with these genetic constructs and IC expression was verified 7 days later, using a specific ELISA for the human Fc region (Figure 2C).

IC5 and IC6 (anti-EGFR) were obtained by transient expression of HEK293T cells using plasmid DNA corresponding to the genetic constructs encoding the heavy and light chains in the pFUSE vectors. The supernatants of HEK293T cells grown in suspension and transfected with PEI were evaluated by means of a specific ELISA for the human Fc region, and the presence of the fusion proteins was verified (Figure 2D).

To obtain the mouse ICs, the genes encoding the variable region of the light chain of the rituximab antibodies, or 14F7, were cloned into the vector pFUSE (Ckm) containing the mouse C kappa constant region gene, and thus, the genes encoding the respective light chains were constructed.

In the case of IC7 (anti-CD20), the heavy chain gene was obtained by PCR assembly of the gene segments encoding the VH region of rituximab (SEQ ID NO. 2.), the CH region of mouse IgG2a (SEQ ID NO. 15), the connecting peptide (Gly₄Ser)₃ThrGly (SEQ ID NO. 44) and of the IL2non-alpha molecule (SEQ ID NO. 21). The resulting heavy chain gene and the light chain gene contained in the pFUSE vector were amplified by PCR and independently cloned into the pLV-CMV-IRES-Neo vector. In this way, the resulting transfer vectors were used to generate the lentiviral particles further employed in the transduction of CHO-K1 cells. Using a specific ELISA for the mouse Fc region, the IC7 (anti-CD20) fusion protein was detected in the supernatant of transduced CHOK1 cells (Figure 2E).

For IC8 (anti-NGcGM3), the gene segment encoding the IL2non-alpha mutein (SEQ ID NO. 21) fused through its N-terminus to the linker peptide (Gly₄Ser)₃ThrGly was amplified by PCR, and was cloned EcoRV/XbaI into the pFUSE vector (mouse IgG1) previously modified for the insertion of an EcoRV site. In the resulting genetic construct the gene VH14F7, previously amplified by PCR, was cloned EcoRI/NheI. The heavy chain and light chain genes contained in the pFUSE vectors were amplified by PCR and independently cloned into the pLV-CMV-IRES-Neo vector. In this way, the resulting transfer vectors were used to generate the lentiviral particles further employed in the co-transduction of CHO-K1 cells. Using a specific ELISA for the mouse Fc region, the IC8 fusion protein (anti-NGcGM3) was detected in the supernatant of transduced CHOK1 cells (Figure 2E).

### Example 2. ICs are expressed as intact and functional proteins.

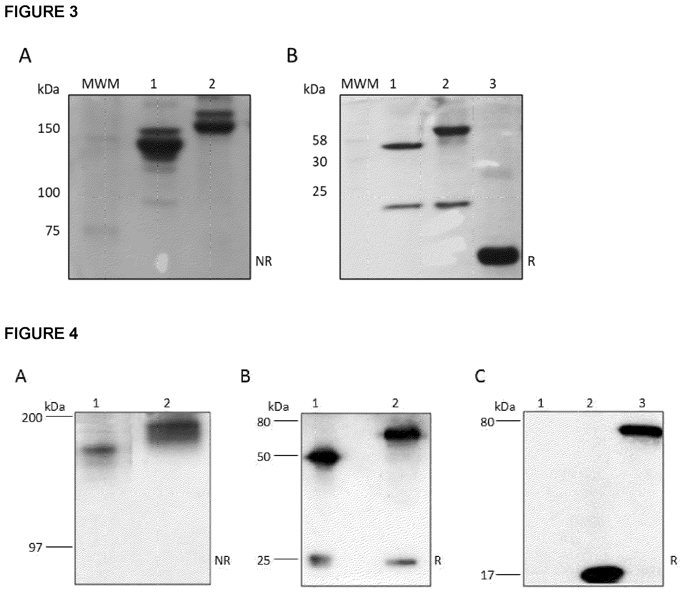

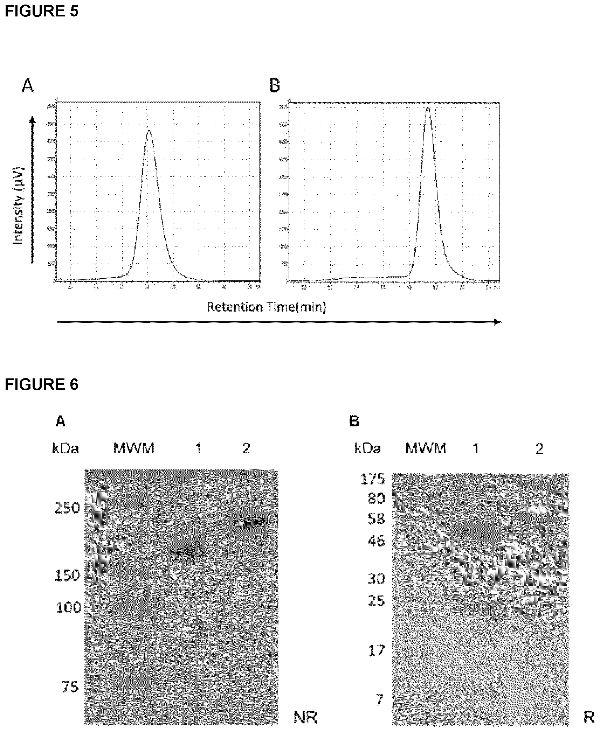

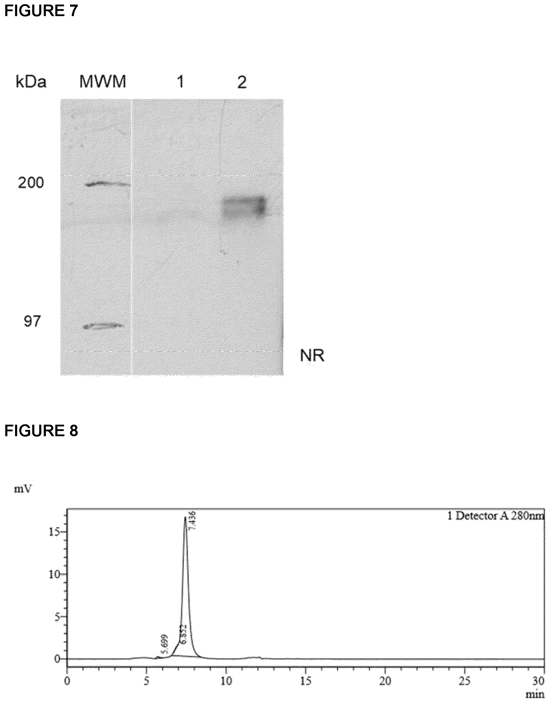

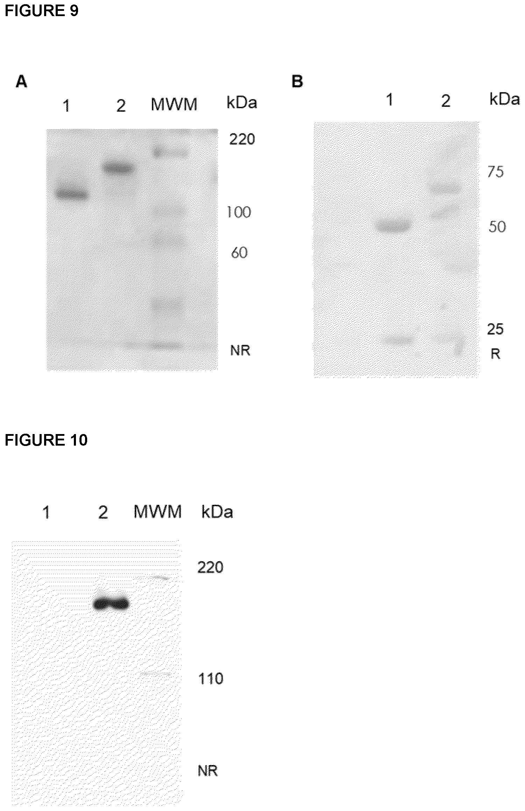

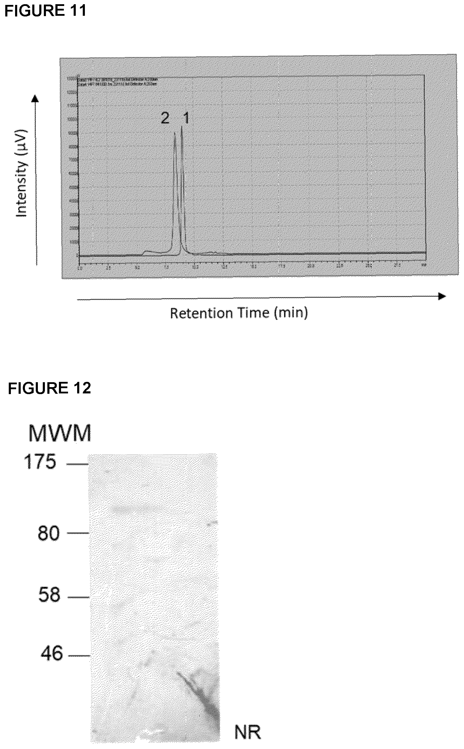

From the culture supernatants of the recombinant CHO-K1 clones, we proceeded to purify the ICs anti-CD20-IL2non-alpha (IgG1h) (IC1), anti-CD20-IL2non-alpha (mouse IgG2a) (IC7) and anti-NGcGM3-IL2non-alpha (mouse IgG1) (IC8). A protein A capture step and subsequent molecular exclusion chromatography on a preparative scale were carried out, using gel filtration. Then, the size and immunochemical identity of the purified proteins were evaluated. It was verified that the ICs were expressed as proteins whose electrophoretic migration corresponds to the approximate theoretical size of 180 kDa, for non-reducing conditions (Figure 3A, Figure 6A and Figure 9A), and in reducing conditions the presence of both chains of the recombinant molecules with a migration corresponding to the theoretical sizes was observed: approximately 65 kDa for the heavy chain, due to the presence of the IL2non-alpha mutein (15kDa), and 25 kDa for the light chain (Figure 3B, Figure 6B and Figure 9B). The identity of the antibody moiety was verified by performing a Western blot assay specific for human IgG (Figure 4A-B and Figure 12), whereas the presence of the cytokine in the structure of the ICs was proved by the same technique with a specific antibody for human IL-2 (Figure 4C, Figure 7 and Figure 10). The purity of the fusion proteins was analyzed by SEC-HPLC on an analytical scale, with a TSK3000 column (Figure 5A, Figure 8 and Figure 11). Because they have a higher molecular weight compared to an IgG antibody (150kDa), they showed a shorter retention time than that of reference mAbs (Figure 5A-B, and Figure 11). A high percentage of monomeric species (>95%) was also observed, indicative of their high purity (Figure 5A, Figure 8 and Figure 11).

### Example 3: ICs preserve and enhance in vitro the biological activities corresponding to the antibody moiety.

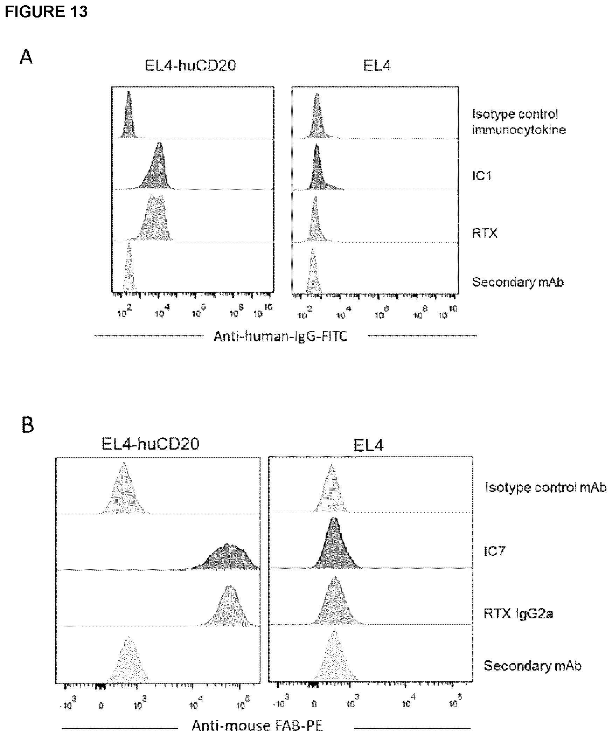

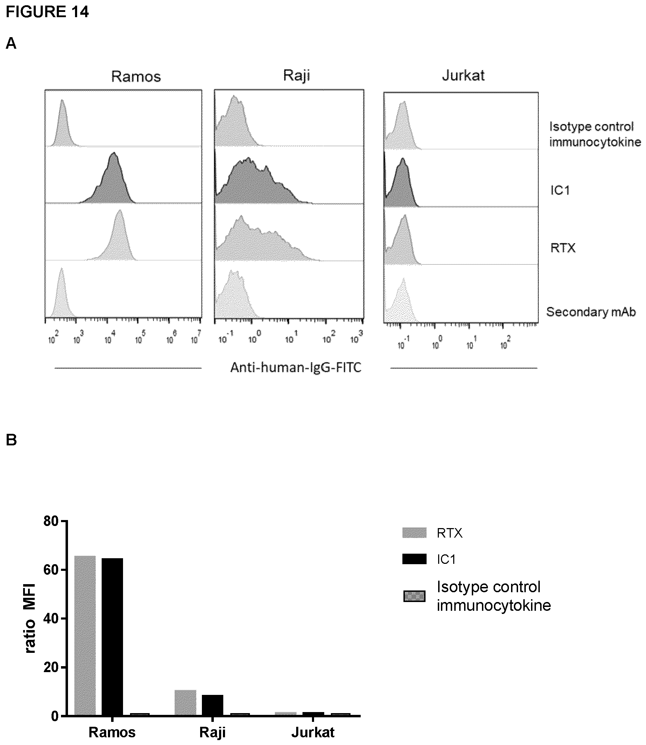

To determine if the antibody portion was active in the structure of the obtained anti-CD20 ICs, in vitro experiments were performed using equimolar amounts with respect to the parent or reference molecule. The recognition of the human CD20 molecule by the ICs anti-CD20-IL2non-alpha (IgG1h) (IC1) and anti-CD20-IL2non-alpha (IgG2a mouse) (IC7) was determined by flow cytometry, using the murine tumor cell line EL4-huCD20, which expresses high levels of human CD20 on its membrane (Di Gaetano, N., et al. (2003) J Immunol. 171(3): 1581-1587). The levels of recognition of EL4-huCD20 cells by IC1 and rituximab (positive control) as well as of IC7 and its parental antibody (Rtx-IgG2a) were similar (Figure 13A-B). The above results demonstrate the ability of both ICs to bind to and recognize the human CD20 molecule. Recognition of the EL4 parental cell line with the ICs was negative, which supports the specificity of the labeling observed on the EL4-huCD20 mouse lymphoma cell line (Figure 13A-B). In addition, two human cell lines of Burkitt's Lymphoma, which express CD20, were evaluated. No differences in the recognition pattern of IC1 compared to rituximab were seen for each of these lines, as Figures 14A and B indicate. The specificity of the label is supported by the non-recognition of an irrelevant IC and the inability of IC1 to bind to Jurkat cells, CD20 non-expressing T lymphoma (Figure 14A-B).

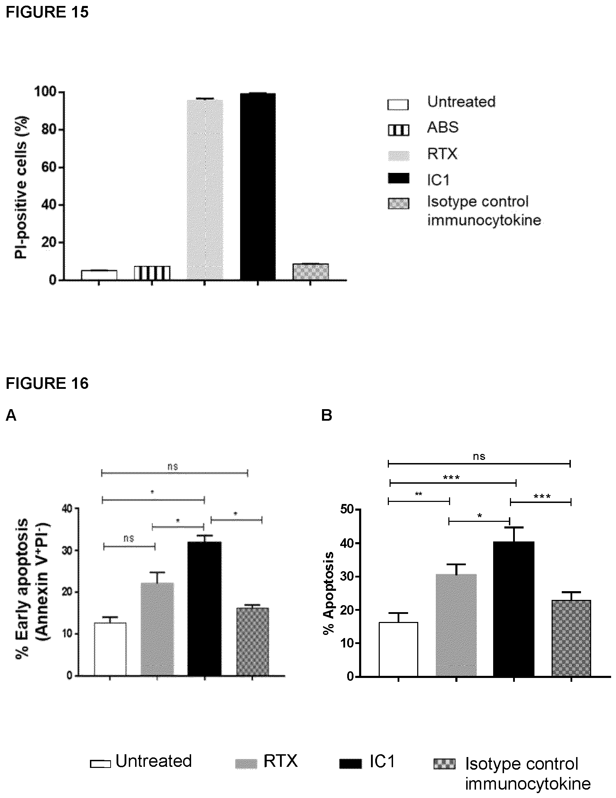

Activation of the complement cascade is one of the proposed mechanisms for the *in vivo* therapeutic activity of rituximab (Smith, M. R. (2003) Oncogene. 22(47): 7359-7368; Seyfizadeh, N., et al. (2016) Crit Rev Oncol Hematol. 97: 275-290). For this reason, the ability of IC1 to exert CDC on the Ramos tumor cell line was evaluated by flow cytometry, through the incorporation of Propidium Iodide (PI). Human AB serum at a final dilution of 1:5 was used as a source of complement. The average values ± standard deviation of percentage (%) of positive PI cells obtained for three independent samples were represented (Figure 15). IC1 at 6nM retained the cytotoxic effect observed with rituximab.

The irrelevant IC did not induce cytotoxicity at the tested concentration (Figure 15). This result shows that this activity is not affected for this molecule, as it has occurred for other ICs of a similar format (Gillies, S. D., et al. (2005) Blood. 105(10): 3972-3978; Gillies, S. D., et al. (1999) Cancer Res. 59(9): 2159-2166).

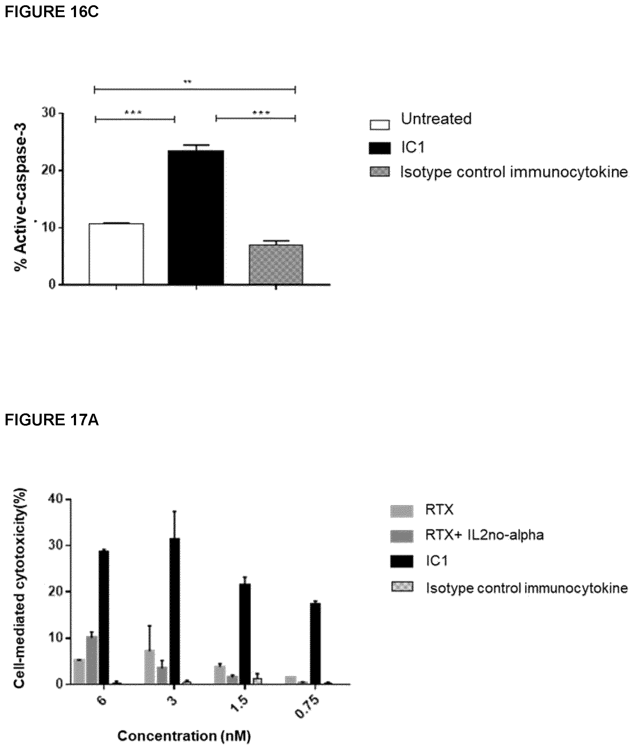

Apoptosis on tumor cells is another of the mechanisms proposed to explain the therapeutic action of rituximab in the clinics (Smith, M. R. (2003) Oncogene. 22(47): 7359-7368; Seyfizadeh, N., et al. (2016) Crit Rev OncolHematol.97: 275-290). Phosphatidylserine exposure is an event that characterizes early stages of apoptosis (Koopman, G., et al. (1994) Blood. 84(5): 1415-1420). The exposure of this phospholipid in the outer monolayer of the plasma membrane was evaluated in Ramos cells treated with IC1. The phosphatidylserine level was measured by flow cytometry by labeling with Annexin V. The average values ± standard deviation obtained for three samples were represented. Cells in early apoptosis were identified as those that presented the Annexin V+/PI- labeling (Figure 16A); cells in late apoptosis were those Annexin V+/PI+ labeled, and apoptosis was defined as the sum of these two populations (Figure 16B). Surprisingly, IC1 had a superior effect than rituximab (Figure 16A-B). Taking into consideration that phosphatidylserine exposure can occur in non-apoptotic events, caspase 3 activation as a marker of late apoptosis was further evaluated. The 17/19 kDa active fragment of caspase 3 was determined by flow cytometry with a specific rabbit serum and an Alexa Fluor-488 conjugated rabbit antiserum. The average values ± standard deviation of percentage of active caspase 3 cells obtained for three independent samples were represented. Incubation of Ramos cells with IC1 at 6nM for 48 h induced activation of this enzyme (Figure 16C).

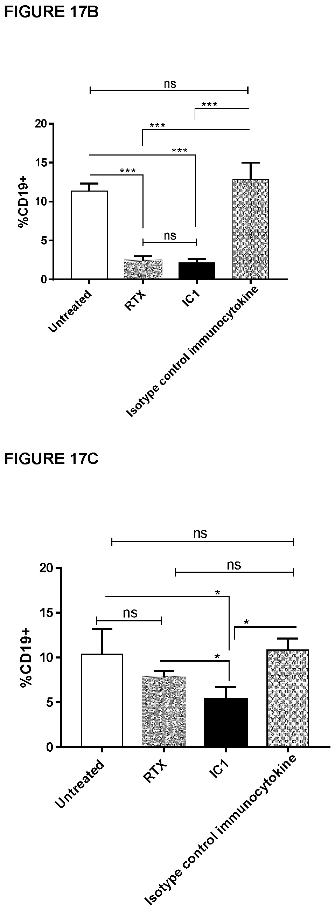

ADCC is another of the mechanisms responsible for the antitumor action of rituximab (Smith, M. R. (2003) Oncogene. 22(47): 7359-7368; Seyfizadeh, N., et al. (2016) Crit Rev OncolHematol. 97: 275 -290). The capacity of IC1 to mediate ADCC was determined through the evaluation of the activity of the LDH enzyme released by the target cells (Ramos), as a measure of the cell damage. This experiment was done in a suboptimal setting for rituximab in terms of effector cell: target cell ratio (10:1), and the use of non-activated effector cells. Peripheral blood mononuclear cells (PMBC) were used as effector cells and Ramos, as target cells. As shown in Figure 17A, IC1 induced a higher percentage of specific lysis with respect to rituximab and the combination of rituximab and IL2non-alpha. The induced cytotoxicity was concentration dependent.

Considering the superiority of IC1 in the induction of ADCC on Ramos cells, its ability to eliminate, in vitro, malignant cells (CD19+) from a Non-Hodgkin Lymphoma patient was studied, with PBMC being the source of target and effector cells. In this experiment, the PBMCs were incubated with the ICs and rituximab (18nM) for 24 hours and the percentage of B cells within the PBMCs was measured by flow cytometry. As shown in Figure 17B, IC1 and rituximab were able to kill normal B cells from a healthy donor. Notably, only IC1 significantly reduced the percentages of CD19+ cells within the PBMCs of a Diffuse Large B-cell Lymphoma patient (Figure 17B). This indicates the possibility of using this IC1 in patients whose resistance to rituximab is related to failure or non-optimal ADCC.

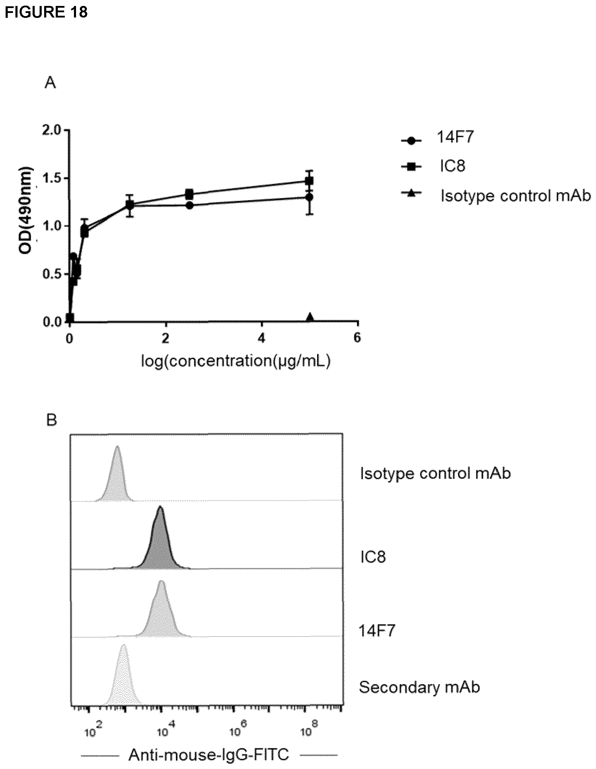

Mouse immunocytokine anti-NGcGM3-IL2non-alpha (IC8) recognized the ganglioside NGcGM3, by ELISA, similarly to the parental antibody 14F7. The specificity of the binding is supported by the non-reactivity of the irrelevant mAb used as isotype control (Figure 18A). As a complement to the solid phase immunoenzymatic assay, a flow cytometry experiment was carried out. L1210 mouse tumor cells were used, which express high levels of the ganglioside NGcGM3 (Roque-Navarro, L., et al. (2008) Mol Cancer Ther. 7(7): 2033-2041). IC8 was able to bind to this tumor cell line, as does the 14F7 antibody (Figure 18B).

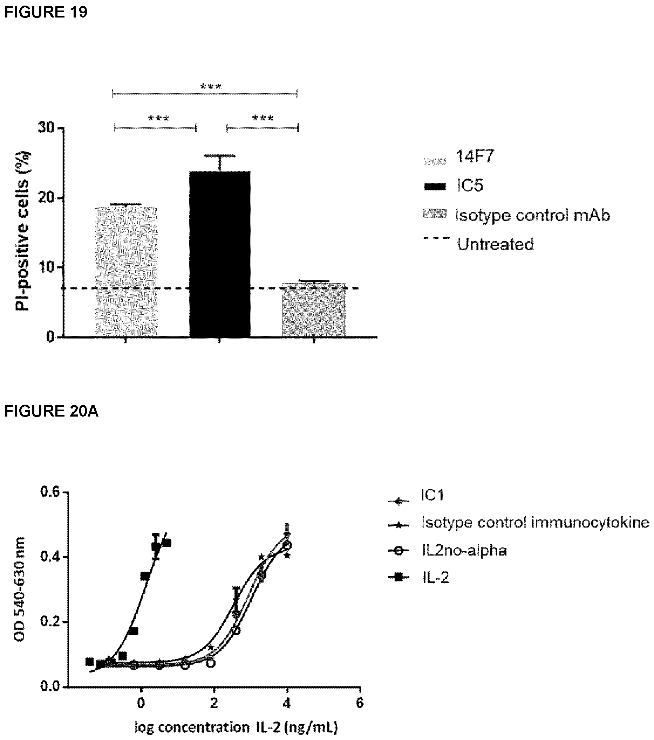

Next, we proceeded to evaluate the cytotoxic activity independent of effector functions in L1210 cells, previously described for 14F7 (Roque-Navarro, L., et al. (2008) Mol Cancer Ther. 7(7): 2033-2041). For this, the increase in membrane permeability to nuclear DNA labeling with PI was analyzed as an index of cell death. Surprisingly, the percentage of non-viable cells was higher for IC anti-NeuGcGM3-IL2non-alpha relative to cells without treatment and parental antibody (Figure 19).

### Example 4. ICs retain the functionality of the non-alpha IL-2 moiety.

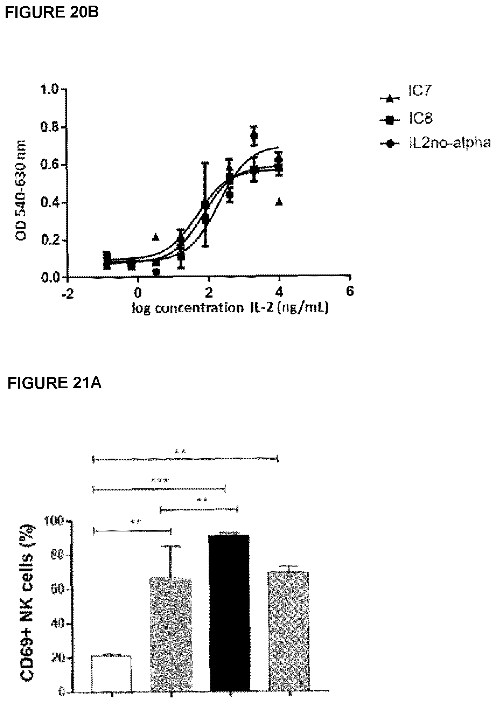

The *in vitro* biological activity corresponding to the IL2non-alpha portion of the ICs was evaluated in proliferation assays of the IL-2-dependent murine cell line CTLL-2, which expresses the high-affinity variant of IL-2R (Figure 20A-B). The concentration values that appear in Figure 19 refer to the non-alpha IL-2 portion contained in each sample.

The incubation of the CTLL-2 cells with the mutein and the ICs, resulted in the stimulation of their proliferation. In all cases, the magnitude of the proliferation presented a concentration-dependent behavior. This result demonstrates that the IL2non-alpha portion maintains its functionality in the context of the fusion proteins (Figure 20A-B).

### Example 5. ICs promote NK Cell Activation

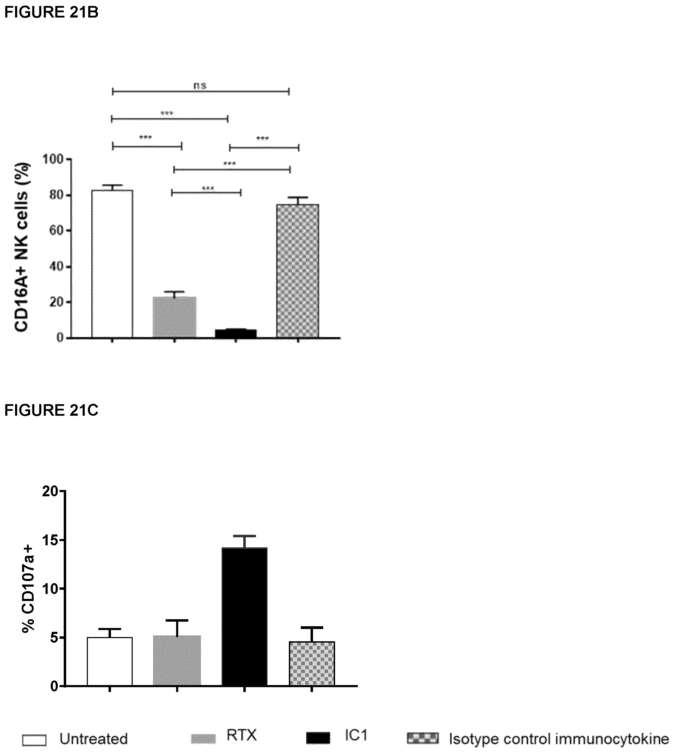

Activation of NK cells is a critical step for ADCC and, furthermore, these cells have been shown to be important mediators of the antitumor function of the IL2non-alpha mutein (Carmenate, T., et al. (2013) J Immunol. 190 (12): 6230-6238). Twenty hours after the assay was set up, the capacity of IC1 to activate NK cells was determined, at 6.6nM, in the presence of target (Ramos) and effector (PMBC) cells, in a 1:10 ratio. In the NK cell population of the samples treated with IC1, a greater decrease in the expression of the surface marker CD 16 was observed as compared to rituximab (Figure 21A), which is indicative of a higher activation of this cell type (Bowles, J.A. and G.J. Weiner. (2005) J Immunol Methods. 304(1-2): 88-99). Likewise, the increase in CD69 expression was verified as an evidence of NK cell activation, an effect also observed in the presence of rituximab (Figure 21B). The expression of CD107a, a surface marker associated with lysosomal degranulation, increased three times in the cells incubated with IC1 with respect to the other treatments (Figure 21C). Taken together, these results demonstrate a greater activation of NK cells associated with ADCC activity in cells treated with this fusion protein, compared to the rituximab antibody, which may probably be due to an enhancing effect provided by IL2non-alpha.

### Example 6. ICs have a superior antitumor effect.

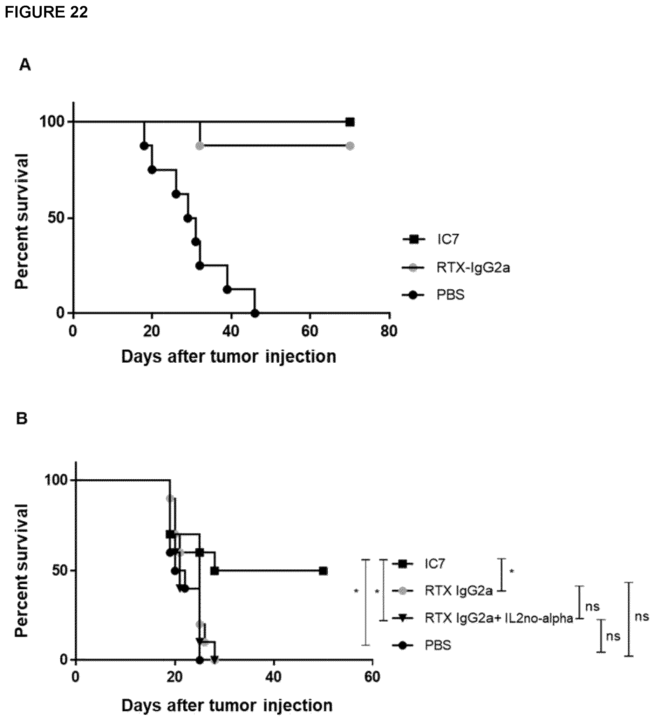

To demonstrate the concept that fusion of non-alpha muteins to an antibody can result in a superior antitumor effect, we compared the antitumor activity of a mouse IgG2a version of rituximab and the corresponding IL2non-alpha-based IC (IC7). For this, C57BL/6 mice were inoculated intravenously with 2.5×10⁵ EL4-huCD20+ cells and subsequently treated intraperitoneally with three injections of 20µg of IC7 (days 1, 4 and 7) or five doses of 200 ug of the parental antibody anti-CD20 IgG2a (days 1, 4, 7, 10 and 13), with demonstrated therapeutic effect in previous investigations (Abes, R., et al. (2010) Blood. 116(6): 926-934; Casadesus, A. V., et al. (2020) Oncoimmunology. 9(1): 1770565.). Interestingly, treatment with IC7 at low doses (20ug) allowed similar survival to that obtained in mice receiving high doses of the IgG2a version of rituximab. (Figure 22A). However, equimolar doses of antibody respect to 20ug of IC7 and its combination with equimolar amounts of the IL2non-alpha mutein had no antitumor effect (Figure 22B).

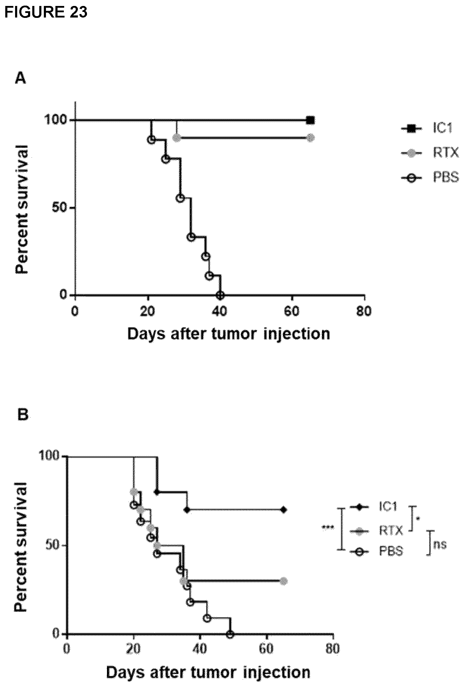

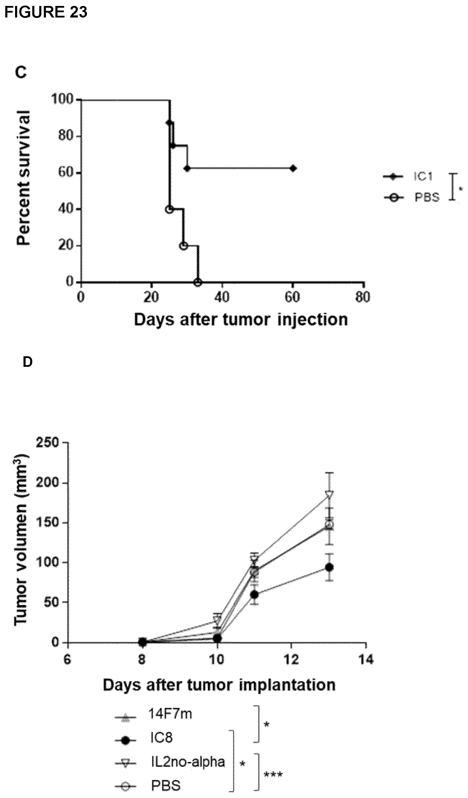

Due to cross-reactivity between human antibodies and mouse effector cells, as well as human IL-2 and mouse IL-2 receptors (Arenas-Ramirez, N., et al. (2015) Trends Immunol. 36 (12): 763-777), we evaluated the efficacy of IC1 in immunocompetent mice. C57BL/6 mice were inoculated intravenously with 5×10⁵ EL4-huCD20 cells and later treated intraperitoneally on days 1, 4 and 7 with 20µg of IC1 and 150ug of rituximab, with a proven therapeutic effect in previous studies (Di Gaetano, N., et al (2003) J Immunol.171(3):1581-1587) (Figure 23A). It was observed that a 7.5-fold lower dose of IC1 compared to rituximab had the same antitumor effect as the antibody (Figure 23A). Furthermore, rituximab in equimolar amounts to IC1 (20ug) had no antitumor effect, unlike the IC (Figure 23B). In addition, after challenging the surviving mice to the previous treatment with IC1 with EL4-huCD20 tumor cells at 65 days, it was verified that IC at low doses maintains the long-lasting protective effect described for a mouse IgG2a variant of rituximab at high doses. (Abes, R., et al. (2010) Blood. 116(6): 926-934) (Figure 23C).

To compare the antitumor effect of anti-NGcGM3-IL2non-alpha (IC8) with respect to controls, C57BL/6 mice were inoculated on day 0 with 2×10⁵ cells of 3LL-D122, in a volume of 200 µL subcutaneously (sc). Six days later, and after verifying its adherence, equimolar amounts of antibody, cytokine, and IC (85 pg for antibodies, 16.6 pg for IL2non-alpha, and 100 pg for IC8) were administered intraperitoneally. Treatments were repeated on days 8 and 10. IC8 was able to reduce tumor volume in treated animals, unlike the control antibody 14F7 (Figure 23D).

### Example 7. ICs preferentially expand NK and CD8+ cells over regulatory T cells.

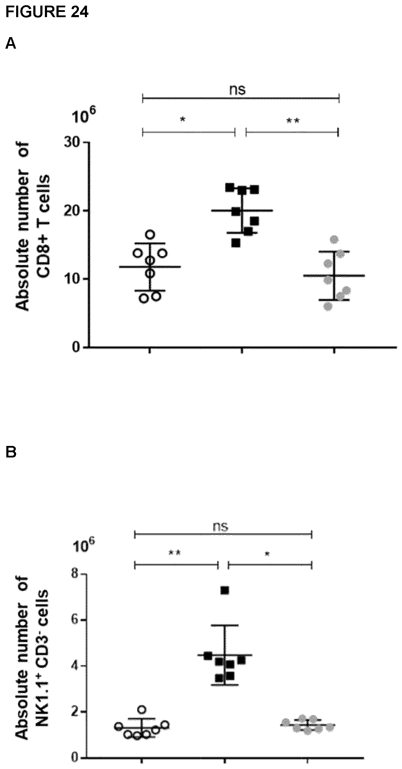

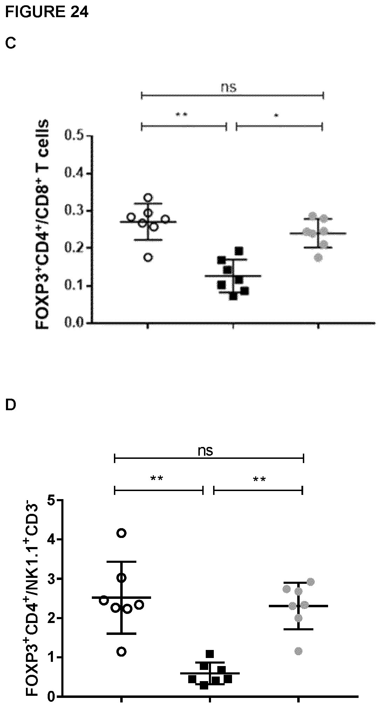

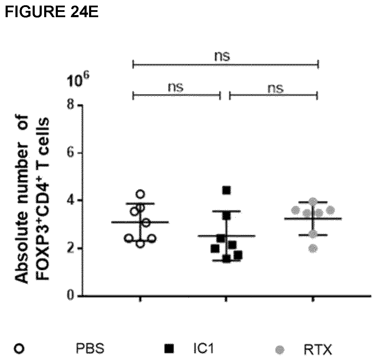

In order to evaluate the effect of non-alpha mutein-based ICs on certain populations of immune cells in the tumor setting, EL4hu-CD20 tumor bearing C57BL/6 mice were treated with anti-CD20-IL2non-alpha (IC1), as described in Example 6. Ten days after tumor challenge, the spleen was removed and different cell populations were analyzed by flow cytometry. For IC1, a significant increase in TCD8+ (Figure 24A) and NK1.1+CD3- (Figure 24B) cells was observed, which was not verified for treatment with rituximab or PBS. It is worth noting that IC1 favored the expansion of effector cells over regulatory T cells, as indicated by the FOXP3+CD4+/TCD8+ and FOXP3+CD4+/NK1.1+CD3- ratio (Figure 24C-D). It is noteworthy that not only the effector cells were more expanded, but no increase in regulatory T cells was observed (Figure 24E). This result demonstrates that IC1 achieves immunomodulation in favor of effector cells, in detriment of regulatory T cells, which could contribute to the superior antitumor effect observed.

Taken together, the previous results point to the therapeutic superiority of an IC based on non-alpha muteins with the previously described format, and indicate that the fusion of anti-CD20 antibodies with proven direct lysis effect, ADCC and/or CDC to non-alpha IL-2 muteins leads to the activation of qualitatively or quantitatively different molecular and cellular mechanisms, which cause an enhanced antitumor response of greater magnitude than that obtained with the controls of antibodies and non-alpha muteins.

## Claims

1. A fusion protein comprising an interleukin 2 (IL-2) agonist mutein linked to an immunoglobulin of the IgG1 or IgG3 isotype and said immunoglobulin is recognized by Fc gamma receptors and complement cascade molecules.

2. The fusion protein according to claim 1 **characterized by** a bivalent molecule with respect to the antigen binding site and the cytokine.

3. The fusion protein according to claim 1 wherein the ability of the mutein to bind to the alpha chain of the IL-2 receptor is affected by at least two orders of magnitude.

4. The fusion protein according to claim 1 wherein the mutein is bound to the immunoglobulin by a linker.

5. The fusion protein according to claim 1 wherein the mutein binds to the heavy chains of the immunoglobulin.

6. The fusion protein according to claim 5 wherein the mutein binds to the carboxy- terminus of the immunoglobulin.

7. The fusion protein according to any of claims 1-6 wherein the mutein has a sequence selected from the group comprising SEQ ID 16-43.

8. The fusion protein according to claim 4 wherein the linker is formed by amino acid sequence selected from the group comprising: (Gly₄Ser)ₙThrGly (SEQ ID NO. 44) and (Gly₄Ser)n (SEQ ID NO. 45), wherein n is the number of repetitions of the fragment Gly₄Ser having a value from 1 to 5.

9. The fusion protein according to claim 1 wherein the IgG1 or IgG3 subclasses comprise the sequence SEQ ID NO. 11 or 12 respectively or have more than 97% identity in respect to said SEQ ID NO. 11 or 12.

10. The fusion protein according to claim 1 wherein the light chain of the immunoglobulin is a human kappa isotype having a sequence that corresponds to SEQ ID NO. 9.

11. The fusion protein according to claim 1 wherein the light chain of the immunoglobulin is a human lambda isotype having a sequence that corresponds to SEQ ID NO. 10.

12. The fusion protein according to claim 1 herein the target tumoral antigen is selected from the group comprising: CD20, CD19, NGcGM3, PDL1, Her1, Her2 and Epcam.

13. A pharmaceutical composition comprising the fusion protein of any of claims 1-12 in a concentration range from 0.5 mg/ml to 20 mg/ml and acceptable pharmaceutical vehicle.

14. Use of the pharmaceutical composition according to claim 13 in the treatment of cancer.

15. Use of the nucleic acid molecule encoding the fusion protein of any of the claims 1-12 for subcutaneous, intramuscular of intratumoral injection.

16. A method of treatment of the subject in need comprising the subcutaneous, intravenous, intradermic, intramuscular or intraperitoneal administration of the pharmaceutical composition of claim 13 in a dose range between 0.01 mg/Kg - 0.6 mg/Kg of body weight.

17. A method of treatment according to claim 16 wherein the administration of the pharmaceutical composition is performed from one to 30 cycles, between one to three doses a week each, spaced seven days to eight weeks apart.
